# EUROPEAN PATENT SPECIFICATION

(11) **EP 2 624 861 B1**
(45) Date of publication and mention of the grant of the patent: **13.05.2020**
(21) Application number: 11770502.0
(22) Date of filing: 07.10.2011
(51) Int. Cl.: A61K 39/015, C07K 16/20

(54) **MALARIA VACCINE**
IMPFSTOFF GEGEN MALARIA
VACCIN CONTRE LA MALARIA

(30) Priority: 08.10.2010 GB 201016969
(43) Date of publication of application: 14.08.2013
(73) Proprietor: Genome Research Limited, London NW1 2BE (GB)
(72) Inventor: WRIGHT, Gavin, J., London Greater London NW1 2BE (GB); RAYNER, Julian, C., London Greater London NW1 2BE (GB); CROSNIER, Cecile, London Greater London NW1 2BE (GB); BUSTAMANTE, Leyla, Y., London Greater London NW1 2BE (GB); BARTHOLDSON, S. Josefin, London Greater London NW1 2BE (GB)
(74) Representative: Mewburn Ellis LLP
(86) International application number: PCT/GB2011/051936
(87) International publication number: WO 2012/046081

(56) References cited:
- WO-A1-2010/022452
- BAUM JAKE ET AL: "Reticulocyte-binding protein homologue 5-An essential adhesin involved in invasion of human erythrocytes by Plasmodium falciparum", INTERNATIONAL JOURNAL FOR PARASITOLOGY, vol. 39, no. 3, February 2009 (2009-02), pages 371-380, XP025896954, ISSN: 0020-7519
- RODRIGUEZ MARILIS ET AL: "PfRH5: A Novel Reticulocyte-Binding Family Homolog of Plasmodium falciparum that Binds to the Erythrocyte, and an Investigation of Its Receptor", PLOS ONE, vol. 3, no. 10, October 2008 (2008-10), XP002670748, ISSN: 1932-6203
- DOUGLAS ALEXANDER D ET AL: "A PfRH5-Based Vaccine Is Efficacious against Heterologous Strain Blood-Stage Plasmodium falciparum Infection in Aotus Monkeys", CELL HOST & MICROBE, vol. 17, no. 1, January 2015 (2015-01), pages 130-139, ISSN: 1931-3128
- BUSTAMANTE LEYLA Y ET AL: "A full-length recombinant Plasmodium falciparum PfRH5 protein induces inhibitory antibodies that are effective across common PfRH5 genetic variants", VACCINE, vol. 31, no. 2, January 2013 (2013-01), pages 373-379, ISSN: 0264-410X(print)
- DOUGLAS ALEXANDER D ET AL: "The blood-stage malaria antigen PfRH5 is susceptible to vaccine-inducible cross-strain neutralizing antibody (vol 2, pg 601, 2011)", NATURE COMMUNICATIONS, vol. 4, September 2013 (2013-09), page Article No.: 2355, ISSN: 2041-1723

## Description

### Summary

The present disclosure relates to polypeptides, inhibitors of polypeptide interactions, methods for expressing polypeptides and polypeptides so expressed.

### Background

Parasites of the *Plasmodium* genus are the etiological agents responsible for malaria, a disease mostly occurring in sub-tropical areas and affecting potentially up to 40% of the world population. Amongst the various species that can affect humans, *Plasmodium falciparum* is by far the most virulent, causing over a million deaths annually, mostly in children under the age of five. Despite intensive efforts from the research community, an effective vaccine has yet to be produced. There is currently no approved vaccine for malaria. One vaccine currently in Phase III trials in Africa is RTS,S (produced by GSK). This vaccine targets the liver stages of the parasite life-cycle. Phase II trials with RTS,S have shown between 39% and 59% efficacy, depending on the adjuvant, dose, and clinical end-point used (eg. Asante et al., Lancet Infect Dis, 2011, PMID 21782519; Olutu et al., Lancet Infect Dis, 2011, PMID 21237715). There are ongoing efforts to produce a more effective vaccine for subsequent release.

All of the clinical symptoms of malaria occur during the asexual erythrocytic stage of the parasite life cycle, when the parasite's merozoites invade human red blood cells, replicate and release up to 32 additional merozoites [ref1]. For this reason, and because merozoites are exposed to the host immune system, erythrocytic invasion has been the main centre of attention. Several proteins displayed at the merozoite surface are believed to be critical for invasion and are therefore good vaccine candidates, yet their precise function remains poorly understood. This is largely due to the difficulty in producing large amounts of functional recombinant parasite proteins [ref2]. The high AT content of *Plasmodium* genomes, the high prevalence of low complexity regions in the parasite's proteins, and the difficulty at identifying clear structural domains within these proteins using standard prediction programs are all contributing factors. Production of extracellular proteins, which often contain structurally critical disulfide bonds, adds another level of complexity as correct folding in a heterologous system will only occur in an oxidising environment (typically the secretory pathway of the organism in which the recombinant parasitic proteins are expressed). Membrane-tethered proteins are also difficult to solubilise as they contain hydrophilic ectodomains in close apposition to hydrophobic transmembrane domains [ref 3].

Invasion of the red blood cell requires interaction between proteins displayed on the surface of the merozoite and the red blood cell. As a result almost all merozoite proteins have been previously suggested to be vaccine candidates, based primarily on their location and supposed function. Antibodies which inhibit such interactions have also been suggested for use in immunotherapy. (Baum et al., Int J Parasitol 2009, PMID 19000690). Of all the potential blood stage candidate antigens, two have reached Phase II vaccine trials, MSP1 and AMA 1. In both cases, no protection was observed (AMA 1: Sagara et al., Vaccine 2009, PMID 19874925; Thera et al, N Engl J Med 2011, PMID 21916638; MSP1: Ogutu et al., PLoS One 2009, PMID 19262754). In both cases, the central problem appears to be the inability of the vaccine to protect across multiple strains. AMA 1 in particular is known to induce antibody responses that primarily target the immunizing sequence, and do not cross-protect against other sequences. Like other blood stage proteins, the Rh family (six related proteins in *P. falciparum)* have been suggested for vaccine development, with attempts being made to combine multiple Rh antigens into a single multi-component vaccine (Lopaticki et al., Infect Immun 2011, PMID 21149582).
WO 2010/022452 A1 discloses compositions for treating and preventing malaria using an invasion ligand directed to a protease-resistant receptor.
Cross-protection is widely viewed as the central problem facing blood stage vaccines (Takala and Plowe, Parasite Immunol 2009, PMID 19691559).
The present disclosure relates to polypeptide expression systems, polypeptides and inhibitors for the prevention and/ or treatment of *Plasmodium* infection and/ or disease, and vaccines comprising such polypeptides or inhibitors.

### Statement of invention

The present invention relates to an antibody which binds to CD147, for use in the prevention and/or treatment of *Plasmodial* infection and/or disease.
The present disclosure relates to a method for producing a polypeptide, the method comprising expression of nucleic acid encoding the polypeptide in a eukaryotic cell, and optionally purification of the polypeptide so expressed, wherein:
(i) optionally the expressed polypeptide is not N-glycosylated in the cell
(ii) the nucleic acid encodes an exogenous eukaryotic signal sequence effective to deliver the polypeptide into the secretory pathway of the eukaryotic cell ; and
(iii) the nucleic acid has been codon optimised for expression of the polypeptide in the eukaryotic cell.
The disclosure also relates to a polypeptide expressed by the method of the invention.
The disclosure also relates to a nucleic acid encoding a polypeptide as disclosed herein, operably linked to an exogenous eukaryotic signal sequence effective to deliver the polypeptide into the secretory pathway of a eukaryotic cell and which nucleic acid has been codon optimised for expression of the polypeptide in the eukaryotic cell. Optionally the nucleic acid encodes a polypeptide which contains no N-glycosylation sites.

The disclosure further relates to a vector comprising the nucleic acid, and cell comprising such a vector.

In a further aspect the disclosure relates to an immunogenic composition or vaccine comprising one or more polypeptides or polynucleotides of the invention, or combination of polypeptides and polynucleotides of the invention.

The disclosure also relates to an immunogenic composition or vaccine comprising an Rh5 polypeptide or a fragment or variant thereof for the prevention and/ or treatment of *Plasmodial* infection and/ or disease.

The disclosure also relates to an Rh5 polypeptide or a fragment or variant thereof for the prevention and/ or treatment of *Plasmodial* infection and/ or disease and also relates to an Rh5 polypeptide or a fragment or variant thereof conferring protection across multiple *Plasmodial* strains. In one aspect the Rh5 polypeptide or fragment or variant thereof may elicit an immune response which is capable of recognizing the same Rh5 polypeptide or Rh5 polypeptides having different sequences, which may be natural variant sequences.

The disclosure also relates to an anti-Rh5 antibody, or fragment or derivative thereof, for use in the prevention or treatment of malarial disease and/or *Plasmodial* infection, and to an anti-Rh5 antibody, orfragment or derivative, thereof capable of preventing red blood cell infection by *Plasmodial* species, for example species which have a different Rh5 sequence to those against which the antibody was raised or otherwise generated.

The disclosure also relates to an inhibitor of the interaction between Rh5 and CD 147, and use of that inhibitor in the prevention or treatment of malaria or malarial infection.

The disclosure also relates to use of a *Plasmodial* polypeptide of the invention in the identification of a red blood cell (RBC) receptor, the method comprising screening red blood cells and/or RBC proteins with the polypeptide to identify RBC components that bind to the malaria polypeptide.

The disclosure also relates to an antibody specifically raised to, or reactive with, a polypeptide produced according to the disclosure. Where the polypeptide of the invention is a fragment of a full length protein (such as an ectodomain) then in one aspect the antibody shows greater specificity to binding of the fragment when compared to the full length protein.

The disclosure further relates to a polypeptide expressed by the method of the disclosure for use in prevention or treatment of disease, such as treatment or prevention of malaria where the polypeptide is a *Plasmodium* polypeptide.

### Figures

**Figure 1****.** Expression of recombinant secreted and cell surface merozoite proteins from *P. falciparum.*
**Figure 2****.** Functional activity and immunogenicity of recombinant *P. falciparum* merozoite proteins.
**Figure 3****.** Demonstration that MSP1 and MSP7 are correctly folded and functional.
**Figure 4****.** Immunogenicity of the recombinant P. falciparum merozoite surface antigens.
**Figure 5****.** AVEXIS identifies two splice variants of the erythrocyte surface protein BASIGIN as a receptor for *P. falciparum* Rh5.
**Figure 6****.** Biophysical characterisation of the Rh5-BSG interaction using surface plasmon resonance.
**Figure 7a** Soluble recombinant ectodomains of BSG-S and BSG-L potently reduce the efficiency of *P. falciparum* erythrocyte invasion.
**Figure 7b****.** Soluble BSG potently block erythrocyte invasion across multiple strains.
**Figure 8a****.** Mouse monoclonal antibodies to human BSG block the invasion of *P. falciparum* into human erythrocytes. Purified monoclonal antibodies MEM-M6/1 (circles) and MEM-M6/6 (squares) were added at the indicated concentrations to an *in vitro P. falciparum* invasion assay using the 3D7 strain. The proteins reduced invasion efficiency relative to an isotype-matched negative control (diamonds)
**Figure 8b**. Anti-BSG antibodies potently block erythrocyte invasion.

### Sequences

### Detailed Description

The present disclosure relates to an expression system for expression of polypeptides. In one aspect the disclosure relates to the expression of polypeptides produced in the malaria parasite, which are generally not glycosylated.

*Plasmodial* proteins are difficult to express [Birkholtz and Blatch "Heterologous expression of Plasmodial proteins for structural studies and functional annotation." Malaria Journal v7 p197 (2008)].

In the studies described, we have attempted to express the 50 entire ectodomain fragments and 3 partial extracellular regions of cell-surface merozoite proteins from *P. falciparum,* using human embryonic kidney (HEK) 293E cells. Using the polypeptide expression system as described in the Example herein we were able to detect expression of 40 proteins by ELISA and 44 by western blot, with 2 showing a lower molecular weight than expected. Thus the disclosure provides a standardised expression system platform suitable for effective expression of a wide range of different polypeptides.

The present disclosure relates to a method for producing a polypeptide, the method comprising expression of nucleic acid encoding the polypeptide in a eukaryotic cell, wherein:
(i) optionally the expressed polypeptide is not N-glycosylated in the cell;
(ii) the nucleic acid encodes an exogenous eukaryotic signal sequence effective to deliver the polypeptide into the secretory pathway of the eukaryotic cell; and
(iii) the nucleic acid is codon optimised for expression of the polypeptide in the eukaryotic cell.

The method may further comprise the steps of purifying the expressed polypeptide, and optionally formulation of the resulting polypeptide with excipients or carriers, or with adjuvants as disclosed herein.

The eukaryotic cell may be a mammalian cell. The signal sequence may be a mammalian signal sequence.

In one aspect the polypeptide is a eukaryotic polypeptide, and in one aspect a *Plasmodial* polypeptide, such as a merozoite polypeptide, such as merozoite surface polypeptide or part thereof. In one aspect the polypeptide is an ectodomain, which is generally a region of a protein that is located outside of the cell. In one aspect the polypeptide is a secreted polypeptide. In one aspect the polypeptide is a cell surface polypeptide. In one aspect the polypeptide is exposed on the surface of a merozoite from *Plasmodium falciparum* strain, such as 3D7, or on the surface of a merozoite from *Plasmodium vivax.*

Polypeptides suitable for expression, in whole or in part, using the method of the disclosure include *Plasmodium* proteins: MSP1, MSP2, MSP4, MSP5, MSP10, Pf12, Pf38, Pf92, Pf113, ASP, RAMA, EBA140, EBA175, EBA181, EBL1, AMA1, MTRAP, MSP3, MSP6, H101, H103, MSP7, Pf41, RhopH3, Rh5, SPATR, TLP, Pf34, PF14_0344, PF10_0323, PFF0335c, AARP, MSP3.4, MSP3.8, MSRP1, MSRP2, MSRP3, RON6, Pf12p, MSP9, GAMA, PF11_0373. In one aspect, the polypeptide is Rh5 or an ectodomain of Rh5 or a fragment or variant thereof. The Rh5 or fragment or variant thereof suitably binds to BASIGIN (CD147), which may be assessed by methods disclosed herein.

Table I lists the accession number for each protein, along with the first and last amino acids of a suitable ectodomain region that may be expressed.

It will be appreciated that in one aspect a polypeptide to be produced will not naturally contain any N-glycosylation sequences. In that case the sequence of the wild type polypeptide, or part of it, may be expressed. However, in another aspect polypeptide sequences will naturally contain N-glycosylation sequences, and in that case the nucleic acid encoding the wild type polypeptide sequence is modified to remove that N-glycosylation sequence from the polypeptide, or the system is arranged in another way to prevent N-glycosylation. These polypeptides are therefore variants of the original (naturally occurring) polypeptide sequence. Therefore, reference herein to polypeptides for expression encompasses variants of those polypeptides in which the polypeptide has been modified when compared to the wild type polypeptide so as to lack any or all N-glycosylation sites, by modification of the nucleic acid encoding the wild type polypeptide. Any reference to polypeptides suitable for use herein includes reference to such variants, where the wild type polypeptide has glycosylation sequences, unless otherwise apparent from the context.

Any suitable polypeptide may be expressed, which may be a naturally occurring polypeptide, or a part or mutant thereof, Mutants include polypeptides which differ in sequence from the naturally occurring sequence by the presence of addition, substitution or deletions. A polypeptide may differ from a naturally occurring polypeptide sequence at 1, 2, 3, 4, 5, 6, 7, 8, 9 or 10, or more amino acids. In one aspect the mutant sequence retains substantially the same function and/or immunogenicity as the naturally occurring wild type sequence. The difference in sequence may be assessed across only the secreted or ectodomain portion of a polypeptide.

Polypeptides suitable for use in the disclosure, such as suitable for expression or other uses as disclosed herein, may be exogenous polypetides which are not encoded by the genome of a mammalian cell but which are found in mammalian cells in nature, suitably in a non glycosylated form. Such polypeptides may be introduced into mammalian cells cell by infection, for example, by a virus, bacteria or other parasite, preferably a eukaryotic parasite. The disclosure is thus not restricted to expression of *Plasmodial* merozoite polypeptides, although these are in one aspect preferred.

In one aspect N-glycosylation within the cell is prevented by provision of nucleic acid encoding a polypeptide which contains no N-glycosylation sites. In this aspect the method comprises modification of the nucleic acid encoding the polypeptide to remove any N-glycosylation sites present in the polypeptide, encoded by Asn-X-Ser or Asn-X-Thr motifs, where X is any amino acid. Methods for the modification of nucleic acid sequences such as site directed mutagenesis are well known in the art. In one aspect the encoded serine or threonine residue in the motif is replaced with a different amino acid residue, such as an alanine residue.

Alternatively the glycosylation of a polypeptide being expressed in the cell is prevented by use of cells in which the N-glycosylation pathway has been inactivated by mutation of one of more of the elements of the glycosylation pathway, and/or the cells are treated with inhibitors of N-glycosylation.

The nucleic acids of the disclosure are codon optimised for expression in the cell type selected for expression. Suitably the codon optimisation is such that expression is optimised for a cell in which the signal sequence is effective and/or is naturally found associated with polypeptides. Codon optimisation may be full or partial optimisation.

The nucleic acids of the disclosure may encode all or part of a naturally occurring polypeptide, or encode a mutant thereof, as above.

The nucleic acid encodes an exogenous eukaryotic signal sequence effective to deliver the expressed polypeptide into the secretory pathway of the cell. In one aspect this signal sequence is from a secreted protein such as an antibody, such as leader sequence of the mouse variable κ light chain 7-33. However, the disclosure is not restricted by this signal sequence and any other suitable leader sequence which directs polypeptides into the cellular secretory pathway may be used.

The signal sequence is operably linked with the nucleic acid encoding the polypeptide to allow the secretion of the expressed polypeptide from the cell.

The signal sequence is exogenous, not being naturally found linked with the polypeptide to be produced. The term 'exogenous' thus refers to the origin of the signal sequence with respect to the polypeptide to be expressed.

In one aspect the polypeptide produced is immunogenic, for example as assessed by the ability to raise an immune response in a human or other mammal.

In one aspect the expressed polypeptide has one, or more, or all, activities of the naturally occurring polypeptide, for example is capable of reacting with antibodies generated by individuals exposed to the naturally occurring polypeptide, or binding to known binding partners, for example as assessed by using methods disclosed herein. In one aspect the binding is of the same affinity as the wild type polypeptide or equivalent fragment of the wild type polypeptide, but may be higher or lower as long as some degree of binding affinity and specificity is observed. Similarly the expressed polypeptide suitably is substantially as immunogenic as the naturally occurring polypeptide, although the immunogenicity may be higher or lower, as long as some degree of immunogenicity is observed.

In one aspect the nucleic acid encodes a polypeptide sequence that acts as a tag, to allow the polypeptide to be easily purified and/or identified. For example, the tag may be a biotinylation sequence, to allow for biotinylated recombinant proteins to be produced in cell culture, and isolated by streptavidin affinity chromatography, or capture of biotinylated proteins on streptavidin-coated solid phases. Other tags and identification/purification systems are well known. The tag is not considered to be a part of the polypeptide of the invention and may comprise a sequence that is glycosylated if cellular conditions allow.

The disclosure also relates to any polypeptide expressed according to the present disclosure, as described above. Suitably the polypeptide is soluble. In particular the polypeptide is an ectodomain of any one of MSP1, MSP2, MSP4, MSP5, MSP10, Pf12, Pf38, Pf92, Pf113, ASP, RAMA, EBA140, EBA175, EBA181, EBL1, AMA1, MTRAP, MSP3, MSP6, H101, H103, MSP7, Pf41, RhopH3, Rh5, SPATR, TLP, Pf34, PF14_0344, PF10_0323, PFF0335c, AARP, MSP3.4, MSP3.8, MSRP1, MSRP2, MSRP3, RON6, Pf12p, MSP9, GAMA, PF11_0373. In one aspect the polypeptide is not secreted from the cell as part of an organelle. In one aspect, the polypeptide is an ectodomain of Rh5, or a fragment or variant thereof.

The disclosure further relates to an isolated nucleic acid encoding a polypeptide which contains no N-glycosylation sites, operably linked to an exogenous eukaryotic signal sequence effective to deliver the polypeptide into the secretory pathway of the cell, which has been codon optimised, suitably for expression in a cell in which the signal sequence is effective.

The disclosure also relates to vectors, such as expression vectors comprising the nucleic acid and cells comprising the expression vector. Suitable vectors include plasmid vectors and viral vectors, as well as transposons. Cells include both bacterial cells, which may be used in standard cloning methodologies, and eukaryotic, such as mammalian cells, in which the nucleic acid is to be expressed.

Previous work on vaccine development involving the Rh family (six related proteins in *P*. *falciparum,* of which Rh5 is one) made attempts to combine multiple Rh antigens into a single multi-component vaccine (Lopaticki et al., Infect Immun 2011, PMID 21149582). Rh5 has been included as a minor component of these studies, but only by expressing small fragments in a bacterial expression system. Importantly, there is no evidence that expressing Rh5 in a bacterial expression system leads to correctly-folded protein.

Rh5 may also be referred to as PfRh5 herein.

The inventors have now shown that red blood cell invasion critically depends on a single receptor-ligand pair between a parasite protein called PfRh5 and a host receptor called BASIGIN (which is also referred to herein as BSG or CD147). BSG has not been previously identified as a receptor used for red blood cell invasion.

The inventors have made the following observations:
1. Blocking the Rh5-BSG interaction with antibodies completely blocks invasion. This puts Rh5 in a different class to many other invasion ligands, including the other Rhs, which are largely redundant, catalysing overlapping pathways. Antibodies against these other proteins will only ever therefore partially block invasion, hence the focus on multi-component vaccine strategies for these proteins.
2. Blocking the Rh5-BSG interaction blocks invasion in all *P. falciparum* strains that we have tested to date rely on the Rh5-BSG interaction, including strains recently isolated from *P*. *falciparum* infected individuals. We have tested 9 laboratory adapted strains, representing 7 different PfRh5 sequences (see Fig 8b(C)) and 6 field isolates (Fig. 8b(D)). This suggests that the Rh5-BSG interaction is universal, and may provide the critical cross-strain protection that has been impossible to generate with other blood stage targets such as MSP1 and AMA1.

The identification of the Rh5-BSG interaction as possibly universal and essential for invasion was unexpected. In one aspect Rh5 may be developed as a single universal target that could allow cross-protection across strains. The advantage of a single component over multi-component vaccine is obviously the lower cost of production - an important consideration for diseases that affect less developed countries.

In particular it has been demonstrated herein that CD147 interacts with Pf Rh5, and that this interaction is involved in invasion. Blocking the interaction using antibodies prevents invasion.

Thus in one aspect the disclosure provides an inhibitor of the interaction of Rh5 and CD147. In another aspect the disclosure relates to an inhibitor of the interaction of Rh5 and CD147 for use in the prevention and or treatment of *Plasmodium* infection and or disease, and in a yet further aspect to the use of the inhibitor in the preparation of a medicament for prevention and or treatment of *Plasmodium* infection and/or malarial disease.

The disclosure also provides a method of prevention or treatment of *Plasmodium* infection or malarial disease, the method comprising delivery of an inhibitor of the interaction of Rh5 and CD147 to an individual in need thereof.

The inhibitor in one aspect is an antibody, which may be a polyclonal or monoclonal antibody, or an antigen-binding derivative or fragments thereof. Well known antigen binding fragments include, for example, single domain antibodies (dAbs; which consist essentially of single VL or VH antibody domains), Fv fragment, including single chain Fv fragment (scFv), Fab fragment, and F(ab')2 fragment. Methods for the construction of such antibody molecules are well known in the art. In one aspect the antibody is humanised.

Modified antibody formats have been developed which retain binding specificity, but have other characteristics that may be desirable, including for example, bispecificity, multivalence (more than two binding sites), and compact size (e.g., binding domains alone). Single chain antibodies lack some or all of the constant domains of the whole antibodies from which they are derived. Therefore, they can overcome some of the problems associated with the use of whole antibodies. For example, single-chain antibodies tend to be free of certain undesired interactions between heavy-chain constant regions and other biological molecules. Additionally, single-chain antibodies are considerably smaller than whole antibodies and can have greater permeability than whole antibodies, allowing single-chain antibodies to localize and bind to target antigen- binding sites more efficiently. Furthermore, the relatively small size of single-chain antibodies makes them less likely to provoke an unwanted immune response in a recipient than whole antibodies. Multiple single chain antibodies, each single chain having one VH and one VL domain covalently linked by a first peptide linker, can be covalently linked by at least one or more peptide linker to form multivalent single chain antibodies, which can be monospecific or multispecific. Each chain of a multivalent single chain antibody includes a variable light chain fragment and a variable heavy chain fragment, and is linked by a peptide linker to at least one other chain. The peptide linker is composed of at least fifteen amino acid residues. The maximum number of linker amino acid residues is approximately one hundred. Two single chain antibodies can be combined to form a diabody, also known as a bivalent dimer. Diabodies have two chains and two binding sites, and can be monospecific or bispecific. Each chain of the diabody includes a VH domain connected to a VL domain. The domains are connected with linkers that are short enough to prevent pairing between domains on the same chain, thus driving the pairing between complementary domains on different chains to recreate the two antigen- binding sites. Three single chain antibodies can be combined to form triabodies, also known as trivalent trimers. Triabodies are constructed with the amino acid terminus of a VL or VH domain directly fused to the carboxyl terminus of a VL or VH domain, i.e., without any linker sequence. The triabody has three Fv heads with the polypeptides arranged in a cyclic, head-to- tail fashion. A possible conformation of the triabody is planar with the three binding sites located in a plane at an angle of 120 degrees from one another. Triabodies can be monospecific, bispecific or trispecific. Thus, antibodies useful in the methods described herein include, but are not limited to, naturally occurring antibodies, bivalent fragments such as (Fab')2, monovalent fragments such as Fab, single chain antibodies, single chain Fv (scFv), single domain antibodies, multivalent single chain antibodies, diabodies, triabodies, and the like that bind specifically with an antigen.

Antibodies can also be raised against a polypeptide or portion of a polypeptide by methods known to those skilled in the art. Antibodies are readily raised in animals such as rabbits or mice by immunization with the gene product, or a fragment thereof. Immunized mice are particularly useful for providing sources of B cells for the manufacture of hybridomas, which in turn are cultured to produce large quantities of monoclonal antibodies. While both polyclonal and monoclonal antibodies can be used in the methods described herein, it is preferred that a monoclonal antibody is used where conditions require increased specificity for a particular protein.
In another aspect the inhibitor is an oligonucleotide (eg DNA or RNA) or peptide aptamer which can bind either a polypeptide made according to the present invention, or the binding target of a polypeptide made according to the present invention, and/or which can prevent interaction of the wild type equivalent of a polypeptide of the invention with its target red blood cell receptor.
In one aspect the inhibitor, such as an aptamer or an antibody or derivative or fragment thereof, binds to CD147.
In one aspect the inhibitor, such as an aptamer or an antibody or derivative or fragment thereof binds to a merozoite target from *Plasmodium falciparum* such as Rh5, or equivalent protein in other *Plasmodium* species.
Thus the invention in particular relates to any anti-CD147 antibody, such as Metuximab, for use in the prevention and/or treatment of *Plasmodial* infection and/ or
malarial disease. The disclosure also describes any anti-Rh5 antibody, for this use.
In one aspect the antibody is an anti-CD147 (also called HAb18G) antibody which has been licensed for use to treat hepatocellular carcinoma by Chinese State Food and Drug Administration (No. 820050039). It is a radiolabelled F(ab)'2 called Licartin (metuximab-l131).

In one aspect the antibody is an antibody that is capable of competing with MEM-M6/6 or TRA-1-85 for binding to CD147.
In one aspect the antibody is a humanised version of either of the antibodies MEM-M6/6 or MEM-M6/1. (MEM-M6/1) was purchased from AbD-Serotec and purified using protein G columns (GE Healthcare) using standard procedures.
In one aspect, the invention relates to a humanized anti-CD147 antibody.
In one aspect the disclosure relates to antibodies against *Plasmodium falciparum* Rh5.
In one aspect the antibody is a humanised version of any anti-Rh5 polypeptide or fragment or variant thereof.
In one aspect the inhibitor of binding is a small molecule, which binds to either the red blood cell (e.g. to CD147) or to a *Plasmodial* target (eg Rh5) to prevent interaction, for example to prevent Rh5 and CD147 interaction.
In a further aspect the inhibitor may be a soluble fragment of one of Rh5 or CD147, which has been shown to help prevent invasion of *P. falciparum in vitro* herein, such as a fragment generated by the methods disclosed herein.

For the avoidance of doubt the inhibitor need not be restricted to *P falciparum* Rh5, but may be an inhibitorfrom any *Plasmodium* species.

Furthermore, for the avoidance of doubt, Rh5 may be expressed in any suitable form, by any method, not limited to that disclosed specifically herein, and may be expressed without any protein sequence modification (e.g. with the native signal sequence and glycosylation sites). In one aspect the Rh5 polypeptide or sequence or variant thereof is expressed with a native signal sequence. In one aspect the Rh5 polypeptide is expressed in a mammalian expression system.

The disclosure also relates to an Rh5 polypeptide or a fragment or variant thereof for the prevention and/ or treatment of *Plasmodial* infection and/ or disease. In one aspect, the disclosure relates to an Rh5 polypeptide or a fragment or variant thereof for conferring protection across multiple *Plasmodial* strains.

In a yet further aspect the disclosure relates to the use of an Rh5 polypeptide or a fragment or variant thereof in the preparation of a medicament for prevention and or treatment of *Plasmodium* infection and/or malarial disease.

In a yet further aspect the disclosure relates to the use of the Rh5 polypeptide or a fragment or variant thereof in the preparation of a medicament for conferring protection across multiple *Plasmodial* strains or species.

Thus in one aspect the disclosure relates to the use of an Rh5 polypeptide or a fragment or variant thereof from a first *Plasmodium* strain in the manufacture of an immunogenic composition capable of preventing *Plasmodium* infection or related disease by a different *Plasmodium* strain or species, such as one having a different Rh5 polypeptide sequence.

The disclosure also relates to a method of prevention or treatment of *Plasmodium* infection or malarial disease, the method comprising delivery of Rh5 polypeptide or a fragment or variant thereof to an individual in need thereof.

The disclosure also relates to a method of conferring protection across multiple *Plasmodial* strains, or species, the method comprising delivery of Rh5 polypeptide or a fragment or variant thereof to an individual in need thereof.

The disclosure also relates to an immunogenic composition or a vaccine comprising the Rh5 polypeptide or a fragment or variant thereof. The immunogenic composition or vaccine may comprise additional antigens, such as *Plasmodium* antigens CSP, MSP-1, AMA1, or part thereof. In one aspect, the immunogenic composition or vaccine does not comprise additional antigens. In one aspect, the immunogenic composition or vaccine does not comprise other members of PfRh and/or EBL families.

In one aspect, the Rh5 or a fragment or variant thereof is expressed using an expression system and method as described herein, preferably using a mammalian expression system, but is not limited to being expressed in this way. In one aspect the Rh5 polypeptide is expressed in a mammalian expression system.

In one aspect the term "Rh5 polypeptide" refers to a polypeptide comprising, consisting essentially of, or consisting of the amino acid sequence as shown in SEQ ID No. 1, Reference to Rh5 polypeptide includes a polypeptide that comprises SEQ ID No. 1 with an N-terminal signal peptide and a C-terminal rat Cd4 domains 3 and 4 tag.

It will be appreciated that fragments or variants of Rh5, such as additions, substitutions or deletions, which may be naturally occurring, may be used in as immunogenic or vaccine compositions. For the avoidance of doubt, the polypeptide variants of Rh5 are not limited to variants that affect glycosylation.

The Rh5 or fragment or variant thereof used in the disclosure suitably has the ability to bind BASIGIN (CD147). The ability to bind BASIGIN is indicative of correctly-folded protein. Expressing Rh5 using a mammalian expression system as shown herein can demonstrably produce correctly-folded protein as assessed by its ability to bind to BASIGIN. The ability to bind BASIGIN can be assessed using techniques such as surface plasmon resonance. In one aspect, the binding affinity of Rh5 or Rh5 fragment to BASIGIN is similar to or stronger than a KD of 1 µM.

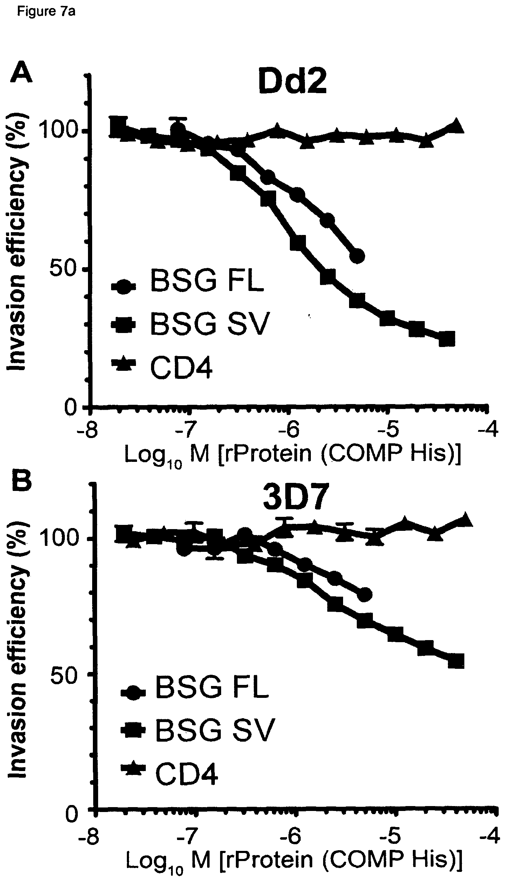

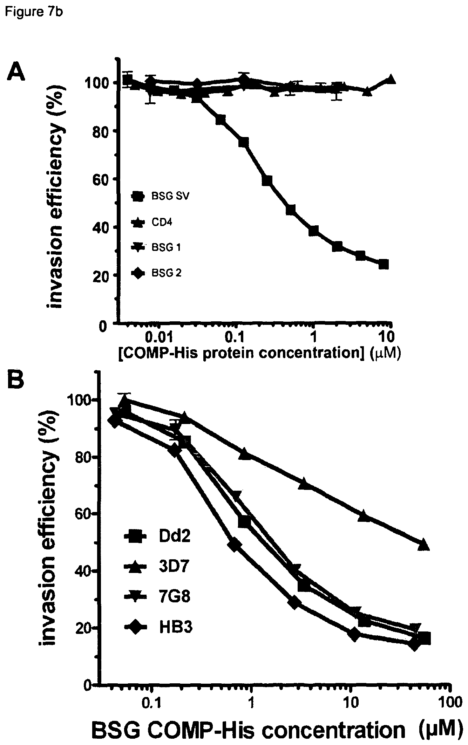

In one aspect of the invention, treatment or prevention of *Plasmodium* infection or malarial disease refers to the complete blocking of invasion of human red blood cells by the *Plasmodium,* for example as assessed by methods disclosed herein. In one aspect, treatment or prevention of *Plasmodium* infection or malarial disease refers to the substantial or significant blocking of invasion of human red blood cells by the *Plasmodium,* for example as assessed by methods disclosed herein. The methods disclosed herein are as described in example 2 and shown in figures 7a and 7b showing inhibition of *Plasmodium falciparum* invasion *in vitro* by blocking the Rh5-CD147 interaction.

In a further aspect the disclosure relates to an immunogenic composition comprising a polypeptide or polynucleotide of the invention. The disclosure also relates to vaccines comprising a polypeptide or polynucleotide of the invention, in particular malaria vaccines using *Plasmodium* antigens expressed using methods described herein.

The immunogenic composition or vaccine may comprise one or more polypeptides or polynucleotides of the invention, or a combination of polypeptides or polynucleotides, preferably a polynucleotide in combination with all or a part of the polypeptide encoded by it, expressed by the methods of the disclosure.

Compositions and vaccines may comprise pharmaceutically acceptable excipients. Suitable excipients are well known in the art and proteins, saccharides, polylactic acids, polyglycolic acids, polymeric amino acids, amino acid copolymers, sucrose (Paoletti et al., 2001, Vaccine, 19:2118), trehalose (WO 00/56365), lactose and lipid aggregates (such as oil droplets or liposomes), diluents, such as water, saline, glycerol, etc. Additionally, auxiliary substances, such as wetting or emulsifying agents, pH buffering substances, and the like, may be present. Sterile pyrogen-free, phosphate buffered physiologic saline is a typical excipient. A thorough discussion of pharmaceutically acceptable excipients is available in reference Gennaro, 2000, Remington: The Science and Practice of Pharmacy, 20.sup.th edition, ISBN:0683306472.

The vaccine of the present disclosure may be used to protect or treat a mammal susceptible to infection, by means of administering said vaccine via systemic or mucosal route. These administrations may include injection via the intramuscular, intraperitoneal, intradermal or subcutaneous routes; or via mucosal administration to the oral/alimentary, respiratory, genitourinary tracts. Thus one aspect of the present disclosure is a method of immunizing a human host against a disease, which method comprises administering to the host an immunoprotective dose of the vaccine or composition of the present disclosure.

The amount of antigen in a vaccine dose is selected as an amount which induces an immunoprotective response without significant, adverse side effects in typical vaccines. Such amount will vary depending upon which specific immunogen is employed and how it is presented. Generally, it is expected that each dose will comprise 10pg - 1mg, such as 1-100ug of protein antigen, suitably 5-50ug, and most typically in the range 5-25ug.

An optimal amount for a particular vaccine can be ascertained by standard studies involving observation of appropriate immune responses in subjects.

Following an initial vaccination, subjects may receive one or several booster immunisations adequately spaced.

Following an initial vaccination, subjects may also receive further vaccinations with antigens which are different from the initial vaccine, for example containing a polypeptide which is naturally occurring but with a different sequence, or is a mutant or variant of a wild type sequence.

The immunogenic composition or vaccine may comprise additional antigens, such as *Plasmodium* antigens, for example those, comprising CSP, for example RTSS or AMA1.

The immunogenic composition or vaccine of the disclosure may also comprise suitable adjuvants to increase the immune response to any vaccine. Suitable adjuvants include those inducing either a TH1 or TH2 response, or both, and adjuvants may comprise an aluminium salt, oil in water emulsion, a saponin such as QS21 or an lipid A derivative such as 30-MPL, or combinations thereof, such as the GSK AS01, AS02, AS03, AS04 adjuvant, or the Novartis MF59 adjuvant.

In a further aspect the disclosure relates to the use of polypeptide made using the present disclosure in screening for interactions with a receptor or other binding partner, suitably by exposing the polypeptide to various targets and detecting binding. For example, the disclosure relates to the use of a *Plasmodium* polypeptide of the disclosure in the identification of a RBC receptor, the method comprising screening red blood cells or RBC proteins with the polypeptide to identify RBC components that bind to the soluble polypeptide. The methodology disclosed in Kauth, C.W. et al. [Interactions between merozoite surface proteins 1, 6, and 7 of the malaria parasite Plasmodium falciparum. The Journal of biological chemistry 281, 31517-31527 (2006).] can be used to screen polypeptides against a red blood cells, or RBC extracts, or red blood cell polypeptides. In one aspect the interaction assay herein termed AVEXIS is used, as described in Bushell, Genome Research v18 p 622 (2008).

The same interaction assay approach could be used to introduce specific mutations within proteins and observe the effects of naturally occurring variations on protein function.

Thus the disclosure relates to mutants of the polypeptides of the disclosure, comprising additions or substitutions or deletions, and polynucleotides encoding the same, and to use of such mutants in screening for the effects of variations on polypeptide binding and/or function.

The disclosure also relates to an antibody raised to, or specifically reactive with, a polypeptide produced according to the invention. Where the polypeptide of the disclosure is a fragment of a full length protein (such an an ectodomain) then in one aspect the antibody shows greater specificity to binding of the fragment when compared to the full length protein. Antibodies may be whole antibodies, antibody fragments or subfragments. Antibodies can be whole immunoglobulins of any class e.g. IgG, IgM, IgA, IgD or IgE, chimeric antibodies or hybrid antibodies with dual specificity to two or more antigens of the disclosure. They may also be fragments e. g. F (ab') 2, Fab', Fab, Fv and the like including hybrid fragments. An immunoglobulin also includes natural, synthetic or genetically engineered proteins that act like an antibody by binding to specific antigens to form a complex.

The disclosure also relates to peptide or nucleic acid (e.g. DNA or RNA) aptamers which bind polypeptides according to the disclosure.

In another aspect the disclosure relates to use of Rh5 or a fragment or variant thereof in identification of an Rh5 ligand suitable for the prevention or treatment of *Plasmodial* infection or related disease. In another aspect the disclosure relates to use of CD147 or a fragment or variant thereof in identification of an CD147 ligand suitable for the prevention or treatment of *Plasmodial* infection or related disease.

In one aspect prevention or treatment of red blood cell infection by *Plasmodia* is considered as prevention of treatment of *Plasmodial* infection or related disease.

In one aspect the disclosure relates to a cell line expressing PfRh5, suitably a stable cell line. In a preferred aspect the invention relates to a method for producing a merozoite polypeptide ectodomain from *Plasmodium falciparum,* the method comprising expression of nucleic acid encoding the polypeptide in a mammalian human embryonic kidney (HEK) cell, and optionally purification of the polypeptide so expressed, wherein:
(i) (optionally) the expressed polypeptide is not N-glycosylated in the cell;
(ii) the nucleic acid encodes an exogenous mammalian signal sequence from the mouse variable κ light chain 7-33 effective to deliver the polypeptide into the secretory pathway of the mammalian cell ; and
(iii) the nucleic acid has been codon optimised for expression of the polypeptide in the HEK cell.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. In one aspect such open ended terms also comprise within their scope a restricted or closed definition, for example such as "consisting essentially of', or "consisting of".

The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein.

Any element of a disclosure is explicitly contemplated in combination with any other element of a disclosure, unless otherwise apparent from the context of the application.

### Example 1

### A library of functional recombinant Plasmodium falciparum merozoite surface proteins

In this study, we have attempted to express the extracellular domain of 53 secreted or cell-surface merozoite proteins from *P. falciparum,* using human embryonic kidney (HEK) 293E cells. To improve the production of functional recombinant proteins, all coding sequences were codon-optimised for expression in human cells and any potential N-linked glycosylation site modified so as to more closely mimic the shape of the native protein. Endogenous signal sequences were removed and replaced by an exogenous mammalian signal sequence to promote correct addressing of the recombinant proteins to the secretory pathway.

Using this approach, we were able to detect expression of 40 proteins by ELISA and 44 by Western blot. The recombinant proteins were shown to be correctly folded and functional by demonstrating their ability to interact with known binding partners, and by showing their immunogenicity against human sera from malaria-infected patients.

### Material and methods

### Generation of a recombinant merozoite protein library

Sequences encoding the extracellular domains of 53 merozoite cell-surface proteins, with the exception of their signal peptide, were made by gene synthesis (Geneart) and are presented in Table 1. All sequences were codon-optimised for expression into human cells and all potential N-linked glycosylation sites identified by the canonical sequence NXS/T were modified by replacing all serine or threonine residues within the canonical motifs by an alanine residue.

The coding sequences, flanked by unique *NotI* and *AscI* sites, were cloned into a derivative of the pTT3 expression vector 4 between the leader sequence of the mouse variable κ light chain 7-33, and a rat CD4 domains 3 and 4 tag followed by an enzymatic biotinylation sequence as previously described 5. All expression constructs were cotransfected with the BirA biotinylation enzyme into HEK293E cells. The soluble biotinylated recombinant proteins were collected from the cell culture supernatant 6 days post transfection, and dialysed into HBS before analysis.

### ELISA test

The biotinylated ectodomains of the *P. falciparum* library were serially diluted 1:2 up to a final dilution of 1:128 and all dilutions were immobilized on streptavidin-coated plates (NUNC) before being incubated for one hour with 10 µg/ml OX68 antibody, which binds the CD4 tag. The plates were washed in PBS/ 0.1% Tween20 (PBST) before incubation with an anti-mouse immunoglobulin antibody coupled to alkaline phosphatase (Sigma) for one hour at room temperature. After washes in PBST and PBS, wells were incubated with p-nitrophenyl phosphate at 1 mg/ml and optical density measurements (OD) taken at 405nm.

### Western blot

Between 5 and 30 µL of dialysed transfection medium containing the recombinant proteins was resolved by SDS-PAGE under reducing conditions (with the exception of EBA181 and EBL1 which were run in non-reducing condition) before blotting onto Hybond-P PVDF membrane (GE Healthcare) overnight at 30 V. Membranes were blocked with 2% BSA in PBST and incubated with 0.02 µg/ml of streptavidin-HRP (Jackson Immunoresearch) diluted in 0.2% BSA and detected with the Supersignal West pico chemiluminescent substrate (Pierce).

Interaction between MSP1 and MSP7 proteins was identified using the AVEXIS method as previously described 5. Briefly, the codon-optimized sequence for MSP1 and MSP7 was cloned into a prey construct between the leader sequence of the mouse variable κ light chain 7-33, and a rat CD4 domains 3 and 4 tag followed by the pentamerisation domain of rat cartilaginous oligomeric matrix protein and the betalactamase coding sequence, as previously described 5. MSP1 and MSP7 prey pentamers were screened against the whole biotinylated merozoite library, and positive interactions identified using nitrocefin (Calbiochem). OD measurements were taken at 485nm .

### Flow cytometry

Biotinylated EBA175-CD4d3+4 ectodomains or CD4 domains 3+4 alone (negative control) were immobilized on streptavidin-coated Nile Red fluorescent 0.4-0.6 µm microbeads (Spherotech Inc.) by incubation for 45 min at 4°C and then presented to human erythrocytes. After incubating for 1 hour at 4°C, cells were washed three times in PBS-BSA-NaN3 to remove non-bound beads, resuspended in 1% formalin and analyzed by flow cytometry using an LSR II machine (BD Biosciences). To test for binding specificity, purified human erythrocytes were either treated with 5 mU of *Vibrio cholera* neuraminidase (Sigma) for 1 hour at 37°C and washed twice, or preincubated with the anti-GYPA BRIC 256 monoclonal antibody at a concentration of 0.5 µg/106 cells, prior to incubation with EBA175-coated microbeads.

### Results

The codon-optimized ectodomain sequences of 53 cell-surface and secreted merozoite proteins from *P. falciparum* were co-transfected with the biotinylation enzyme BirA into HEK293E cells (Table 1). Soluble biotinylated recombinant proteins were harvested six days post-transfection and dialysed into HBS. Expression levels of all proteins were first assessed by ELISA: of the 53 proteins tested, all but 12 (RAP1, RAP2, RAP3, RhopH1, RhopH2, RON3, Rh1, Rh2b, Rh4, PF14_0293, EBL1 and PTRAMP) showed clear signals (data not shown).

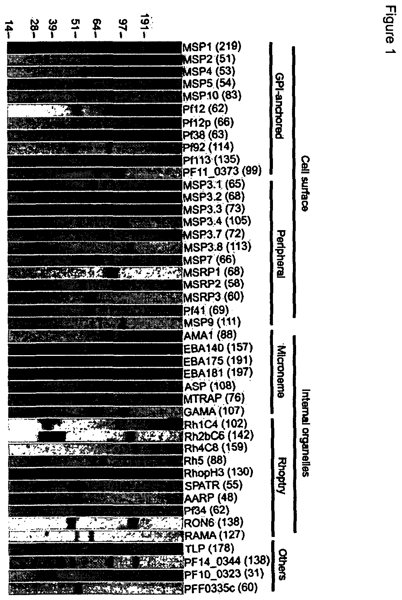

All proteins were then tested by western blot (Figure 1). As expected from the ELISA test, no expression was detected for RAP1, RAP2, RAP3, RhopH1, RhopH2, RON3, EBL1, PF14_0293 and PTRAMP. Most proteins showed expression of one major form.

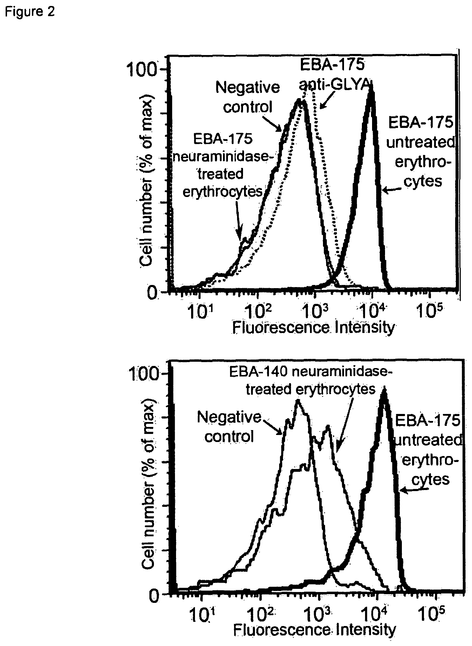

To show that these recombinant proteins were functional, we next assessed their ability to interact with known binding partners. As a first example, we focused on the micronemal protein EBA175, which is known to interact, through its region II, to GLYCOPHORIN A (GYPA) expressed at the surface of human red blood cells 6. This interaction requires sialylation of GYPA as pre-treatment of erythrocytes with neuraminidase, which cleaves off sialic acid residues, is sufficient to abolish binding. To test whether we could recapitulate these observations using our recombinant EBA175 ectodomain, human erythrocytes were presented with Nile Red microbeads coated either with the biotinylated EBA 175 extracellular domain, or the biotinylated CD4 domains 3+4 as a negative control (Figure 2). EBA175-coated but not CD4- coated microbeads bound robustly to human red blood cells. This binding could be specifically blocked by either pre-treatment of erythrocytes with neuraminidase (Figure 2A), or pre-incubation of the red blood cells with an anti-GYPA monoclonal antibody (Figure 2B) demonstrating that the full-length recombinant EBA175 ectodomain could specifically interact with the native GYPA present on the surface of erythrocytes.

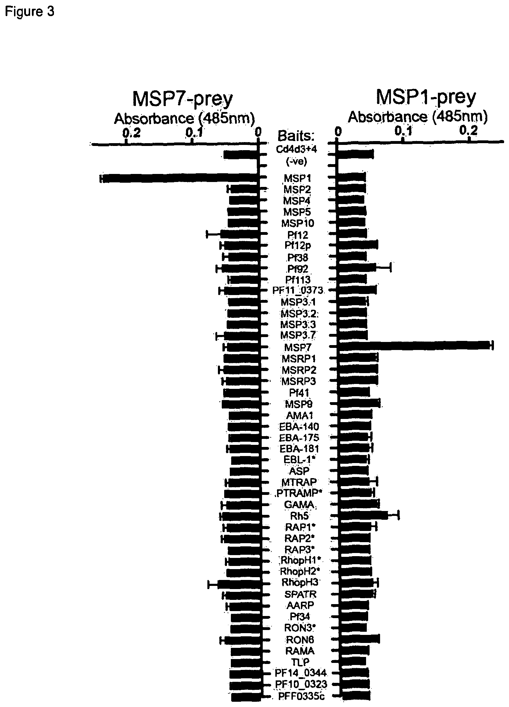

As a second example, we tested interactions between merozoite proteins. The merozoite surface proteins 1, 6 and 7 have previously been shown to form a noncovalent complex at the surface of merozoites, which is subsequently cleaved off upon erythrocyte invasion. All three proteins are believed to undergo proteolytic maturation before forming the complex. Using full-length recombinant MSP7 produced in *E. coli,* Kauth and coworkers (ref 2) demonstrated association with the aminoterminal p83, p30 and p38 subfragments of MSP1, but not with the carboxyterminal p42 region. The p38 fragment of MSP1 was also able to bind the processed carboxyterminal MSP636 form of MSP6. To reproduce binding between these different merozoite surface proteins, we used the AVEXIS method. The codon-optimized sequence of MSP1 and MSP7 ectodomains were cloned into a prey construct and expressed as pentameric proteins fused to beta-lactamase by transfection into HEK293E cells. These 2 preys were then normalised before screening against the whole recombinant merozoite library. Using this approach, we were able to detect the MSP1-MSP7 interaction in both orientations: the MSP1 prey was only captured by the MSP7 bait, and similarly the MSP7 prey was only captured by the MSP1 bait (Figure 3) demonstrating that both proteins were functional. Interestingly, this binding was detected using the whole unprocessed ectodomains of MSP1 and MSP7. No interaction was detected between MSP6 and MSP7. Interestingly, we did not observe any binding between MSP1 and MSP6, confirming the previous observation that only the processed form MSP636 can bind MSP1 (ref 2).

### Example 2: Immunogenicity testing

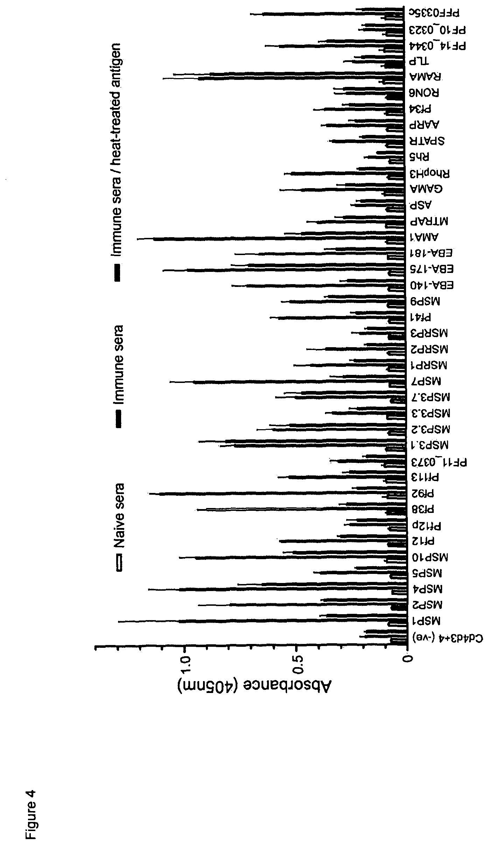

We tested whether any of the recombinant ectodomains were immunogenic by testing them against sera from patients exposed to *Plasmodium falciparum* compared to patients that were malaria naive. This approach has been previously used to provide an indication that *P. falciparum* recombinant proteins are folded correctly. To extend this rationale, we compared the responses of the sera to untreated proteins and those that had been heated for 10 minutes at 80°C with the thinking that many conformational-dependent epitopes would be heat labile. All recombinant proteins gave a higher response to pooled serum from malaria-exposed patients relative to non-exposed controls (Fig. 4). Heat-treatment decreased the reading for all but 9 proteins (Pf12p, MSP3, MSP6, H103, MSRP3, ASP, RON6, RAMA, TLP, PF10_0323) demonstrating that most proteins contain heat-labile epitopes implying that they are correctly folded.

### AVEXIS identifies CD147 as a receptor for PfRh5

One anticipated use of the recombinant *P*. *falciparum* merozoite surface protein library is to identify erythrocyte receptors for the parasite ligands. Here we show how this has been performed for the *P*. *falciparum* merozoite protein, Rh5.

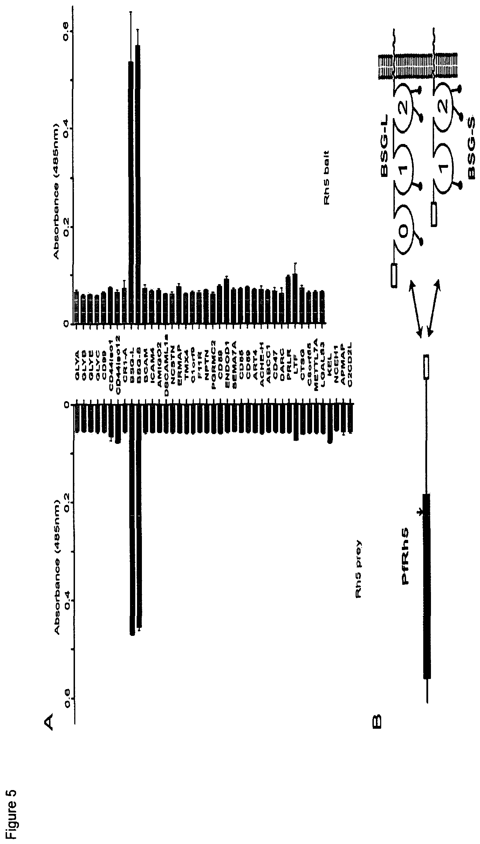

To identify an erythrocyte receptor for the *P*. *falciparum* invasion ligand Rh5, we used a recombinant Rh5 protein taken from the merozoite surface protein protein library. Rh5 was expressed either as a monomeric enzymatically biotinylated "bait" or a beta-lactamase-tagged pentamerised "prey" and their expression activities normalised to a stringent threshold suitable for screening by AVEXIS (7). A protein library produced in an identical fashion but containing the ectodomain regions of human erythrocyte receptors contained 41 baits and 37 preys. The Rh5 prey was screened against the erythrocyte bait library and an interaction with BASIGIN (BSG-L) was detected (Fig. 5A, left panel). The next best interaction was with a shorter splice variant of the same protein, BSG-S. To verify these interactions, the screen was performed in the reciprocal orientation with the erythrocyte prey library screened against the Rh5 bait. We found that both isoforms of the BASIGIN protein interacted with Rh5 (Fig. 5A, right panel). No other interactions with recombinant erythrocyte receptors were detected in our screen.

BASIGIN (also known as CD147, EMMPRIN and M6) is a widely expressed member of the immunoglobulin superfamily (IgSF) (Fig. 5B) that has been implicated in many biological functions ranging from embryo implantation and spermatogenesis (8) to retinal development (9). It has also been implicated in disease processes including tumour metastasis (10), rheumatoid arthritis and human immunodeficiency virus infection. The two IgSF domains belong to the C and V sets but are unusual in that the C-set IgSF domain is located N-terminal to the V-set domain (11). Rh5 interacted with both isoforms of the protein suggesting that the Rh5 binding site resided with the two membrane-proximal IgSF domains.

### Rh5 directly interacts with BSG: a quantitative analysis

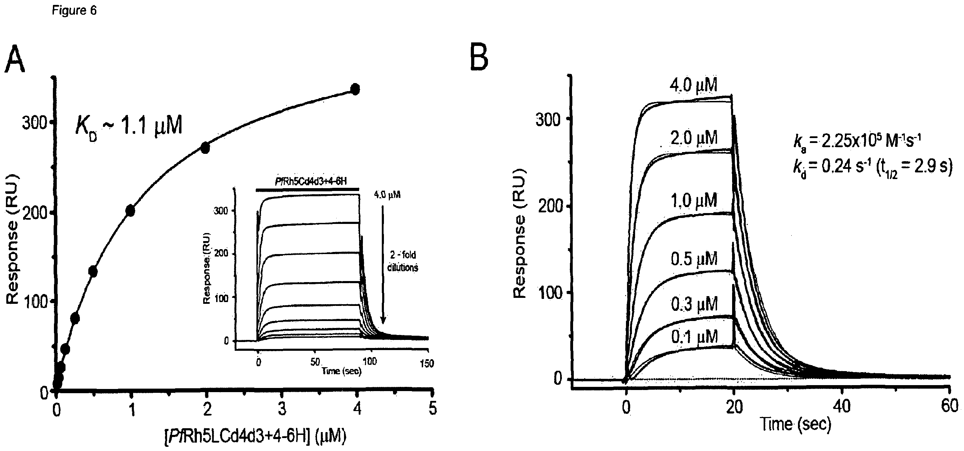

To demonstrate that Rh5 and BSG directly interact and to quantify the binding parameters of the interaction, we used surface plasmon resonance as implemented in a BIAcore machine which is able to detect even very transient interactions. The ectodomain fragment of Rh5 was purified and separated by gel filtration before injecting increasing concentrations over the biotinylated BSG-SCd4d3+4bio immobilised on a streptavidin-coated sensor chip with CD4d3+4bio used as a reference. The binding once equilibrium had been reached (Fig. 6A - inset) is represented by the difference in response units observed in the BSG-S and control flow cell and is plotted as a binding curve (Fig. 6A). Saturable binding was observed and an equilibrium dissociation constant (*K*_{D}) of 1.14 ± 0.03 µM was calculated from a non-linear curve fit to the data.

To determine the kinetic parameters of the interaction, serial dilutions of purified *Pf*Rh5Cd4d3+4-6H were injected over immobilised BSG-S and a reference flow cell at high flow rates (100 µl/min) to minimise rebinding effects. A global fit of a simple 1:1 binding model fitted the curves well (Fig. 6B) and yielded a dissociation rate constant (*k*_{d}) of 0.240 ± 0.001 s⁻¹ (corresponding to an interaction half-life of 2.9 seconds) and an association rate constant (*k*ₐ) of 2.25 ± 0.01 x 10⁵ M⁻¹s⁻¹. The same analysis was performed on the three domain isoform of BSG which showed a slightly stronger interaction strength (*K*_{D} = 0.71 ± 0.02 µM; *k*_{d} 0.1436 ± 0.0003 s⁻¹) suggesting that the additional IgSF domain of the long BSG isoform marginally increased Rh5 binding affinity.

### Soluble BSG-S and BSG-L inhibit P. falciparum invasion in vitro.

To determine whether the interaction between the erythrocyte BSG receptor and *P. falciparum* invasion ligand Rh5 is necessary for invasion, we attempted to specifically block the interaction by adding purified pentamerised recombinant soluble ectodomain fragments of both the long and short forms of human BSG to invasion cultures. We found that BSG-S inhibited invasion of the neuraminidase-sensitive Dd2 *P. falciparum* strain in a dose-dependent manner which had the shape of a typical dose-response curve with an IC₅₀ of ∼1µM (Fig. 7a(A)). This protein also inhibited the invasion of a neuraminidase-independent strain, 3D7, although with reduced efficacy (Fig. 7a(B)). In addition, BSG-L was able to inhibit invasion in both strains (Fig. 7).

Figure 7b shows that soluble BSG potently block erythrocyte invasion across multiple strains.

### Monoclonal antibodies against BSG inhibit P. falciparum invasion in vitro.

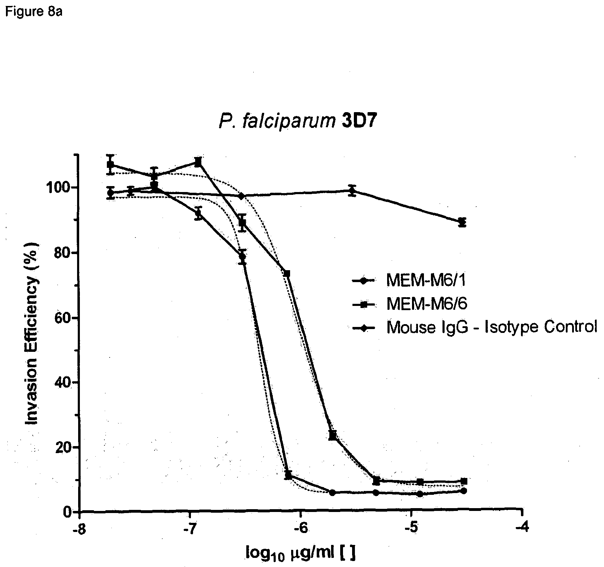

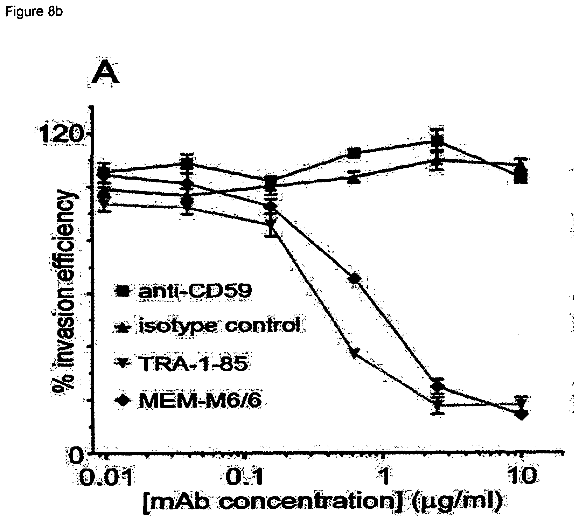

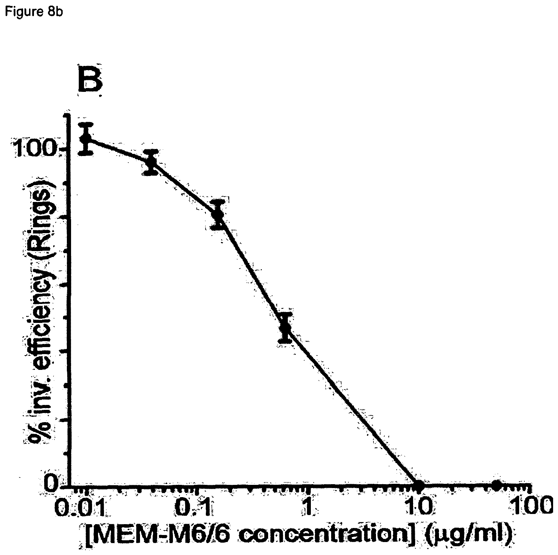

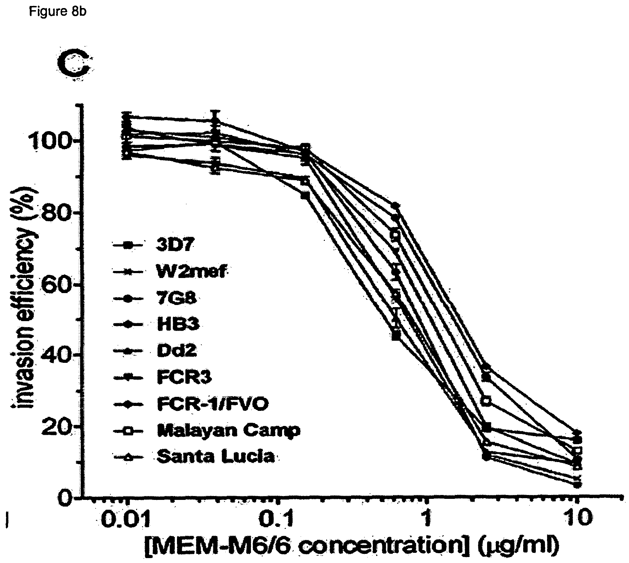

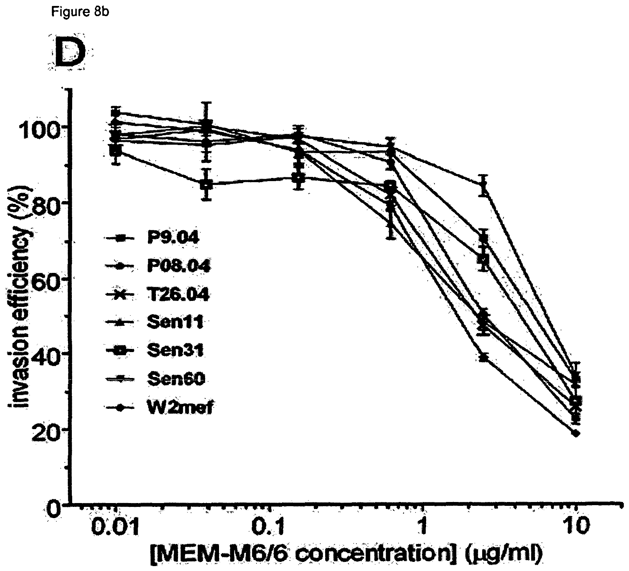

Soluble forms of BSG consisting of the extracellular regions are known to have biological effects such as up regulation of matrix metalloproteases which might indirectly affect erythrocyte invasion efficiency. To rule out this possibility, we added purified monoclonal antibodies which are known to bind human BSG to the in vitro invasion assay. Two independent mouse monoclonal antibodies that recognise human BSG (MEM-M6/6 and MEM-M6/1) both potently blocked invasion (Fig. 8). To our knowledge, this is the first identification of a human - *P. falciparum* receptor ligand pair that is essential for invasion.

### Materials and Methods

### Recombinant protein production

Proteins for inclusion within the human erythrocyte protein library were selected from a comprehensive proteomics analysis of human erythrocyte ghost preparations and included all type I, GPI and type II receptors and secreted proteins. Bait and prey constructs were produced essentially as described (7). Briefly, each construct contained the entire extracellular region (including the native signal peptide) flanked by unique NotI and AscI sites to facilitate cloning into a vector that added a C-terminal rat CD4d3+4-tag and either a enzymatically biotinylatable peptide (baits) or the pentamerising peptide from the rat cartilage oligomeric matrix protein (COMP) followed by the enzyme beta-lactamase (preys). Bait proteins were enzymatically biotinylated during expression by cotransfection of a secreted form of the *E.coli* BirA protein biotin ligase (7). The Rh5 bait and prey constructs differed in that the low-scoring endogenous signal peptide (17) was replaced by a high-scoring signal peptide from the mouse immunoglobulin kappa light chain and potential N-linked glycan sites were mutated. All constructs were codon optimised for mammalian expression and chemically synthesized (Geneart AG, Regensburg, Germany) and subcloned into both bait and prey expression vectors. Monomeric proteins were purified by subcloning the Notl/Ascl flanked extracellular regions into a similar vector encoding a CD4d3+4 tag followed by a hexa-His tag and purified using 1ml HiTrap Ni²⁺ IMAC columns (GE Healthcare) as described (7). Purified pentameric proteins used in invasion assays were made by replacing the beta-lactamase reporter in the prey plasmid with a hexa-his tag and the supernatants from transient transfections purified on HiTrap columns as described above. Purified proteins were then dialysed 3x against PBS and 1x against RPMI prior to use. Individual domains of human BSG were produced by identifying domain boundaries using the structure of the BSG extracellular region (11, 18) and amplifying these domains using primers with suitable restriction cloning sites.

### Interaction screening by AVEXIS

Interaction screening was carried out as described (7).

### Antibodies

Antibodies were purchased from AbD-Serotec (MEM-M6/1) or were a kind gift from Vaclav Horeijsi (Institute of Molecular Genetics, Czech Republic) (MEM-M6/6) and purified using protein G columns (GE Healthcare) using standard procedures.

### Surface plasmon resonance

Surface plasmon resonance studies were performed essentially as described (7, 20) using a BIAcore T100 instrument. Briefly, biotinylated bait proteins were captured on a streptavidin-coated sensor chip (BIAcore, GE Healthcare) using molar equivalents of rat CD4 domains 3 and 4 as a reference. Purified analyte proteins were separated by gel filtration just prior to use in SPR experiments to remove small amounts of protein aggregates which are known to influence binding kinetic binding measurements (21). Increasing concentrations of purified proteins were injected at 10 µl/min for equilibrium studies or 100 µl/min for kinetic analyses to minimise rebinding effects. Binding data were analysed in BIAevaluation software (BIAcore) using a global fits to the entire sensorgrams (both association and dissociation phases) to a dilution series of ligand. All experiments were performed at 37°C.

### In Vitro Culture of P. falciparum Parasites

*P. falciparum* parasite strains 3D7, Dd2, and HB3 were routinely cultured in human O+ erythrocytes (NHS Blood and Transplant, Cambridge, UK) at 5% hematocrit in complete medium containing 10% human sera, under an atmosphere of 1% 02, 3% CO2, and 96% N2 (BOC, Guildford, UK). Parasite cultures were synchronized on early stages with 5% D-sorbitol (Sigma-Aldrich, Dorset, UK). Use of erythrocytes from human donors for *P. falciparum* culture was approved by NHS Cambridgeshire 4 Research Ethics Committee.

### Parasite Labeling

Parasite cultures were stained with a DNA dye according to the following protocol. The cells were washed with PBS before staining with 2 µM Hoechst 33342 (Invitrogen, Paisley, UK) in RPMI 1640. After staining, the cells were washed with PBS, before being fixed with a 2% paraformaldehyde (Sigma-Aldrich, Dorset, UK), 0.2% glutaraldehyde (Sigma-Aldrich, Dorset, UK) solution in PBS for 1 h at 4°C. Finally, the suspension was washed with PBS before acquisition on a flow cytometer. The cells were next washed with PBS before staining with the DNA dyes as described earlier. Finally, the cells were washed with PBS before acquisition on a flow cytometer.

### Erythrocyte Labeling

Erythrocytes were labeled with amine-reactive fluorescent dyes. The required volume of O+ erythrocytes at 2% haematocrit in RPMI 1640 was centrifuged and the pellet resuspended to 2% hematocrit with either 20 µM CFDA-SE (Invitrogen,
Paisley, UK) or 10 µM DDAO-SE (Invitrogen, Paisley, UK) in RPMI 1640 and incubated for 2 h at 37°C. The suspension was washed with complete medium and the pellet resuspended to 2% hematocrit with complete medium and incubated for 30 min at 37°C. The suspension was then washed twice with incomplete medium (without human sera) and finally resuspended to 2% hematocrit with incomplete medium. The cells were stored until use at 4°C for up to 24 h.

### Flow Cytometry and Data Analysis

Stained samples were examined with a 355 nm 20 mW UV laser, a 488 nm 20 mW blue laser, and a 633 nm 17 mW red laser on a BD LSRII flow cytometer (BD Biosciences, Oxford, UK). Ethidium bromide (EB) was excited by a blue laser and detected by a 610/20 filter. Hoechst 33342 was excited by a UV laser and detected by a 450/50 filter. SYBR Green I and CFDA-SE were excited by a blue laser and detected by a 530/30 filter. DDAO-SE was excited by a red laser and detected by a 660/20 filter. BD FACS Diva (BD Biosciences, Oxford, UK) was used to collect 100,000 events for each sample. FSC and SSC voltages of 423 and 198, respectively, and a threshold of 2,000 on FSC were applied to gate on the erythrocyte population. The data collected was then further analyzed with FlowJo (Tree Star, Ashland, Oregon). All experiments were carried out in triplicate and the data is presented as the mean ± standard error of the mean. GraphPad Prism (GraphPad Software, La Jolla, CA) was used to plot parasitemia data generated and carry out statistical analysis.

### P. falciparum invasion assays

Invasion assays were carried out in round-bottom 96-well plates, with a culture volume of 100 µL per well at a hematocrit of 2%. Plates were incubated inside an incubator culture chamber (VWR, Lutterworth, UK), gassed with 1% O₂, 3% CO₂, and 96% N₂, and kept at 37°C for 48 h. Erythrocytes labelled with either 20 µM CFDA-SE (Invitrogen, Paisley, UK) or 10 µM DDAO-SE (Invitrogen, Paisley, UK) were pelleted and washed with incomplete media. The pellet was resuspended to 2% hematocrit with incomplete medium and aliquoted into individual microfuge tubes. Neuraminidase from *Vibrio cholerae* (Sigma-Aldrich, Dorset, UK) was added to the appropriate tubes to obtain a final concentration of 20 mU/mL, and all of the tubes were incubated under rotation at 37°C for 1 h. The cell suspensions were pelleted and washed with incomplete media. The pellets were then resuspended to 2% hematocrit with incomplete medium. pRBC were then added to each well and the well suspension mixed before incubation for 48 h. At the end of the incubation period, RBC were harvested and pRBC were stained as described earlier. Data collection and statistical analysis were carried out as described earlier. Detailed Standard Operating Procedures for all invasion assays are available at http://www.sanger.ac.uk/research/ projects/malariaprogramme-rayner/ (Resources section).

### Immunogenicity to malaria-exposed serum.

The biotinylated ectodomains of the *P. falciparum* library were immobilized on streptavidin-coated plates (NUNC) with or without prior heat denaturation for 10 minutes at 80°C. The concentration of each ectodomain was adjusted so as to obtain saturation of the streptavidin on the well. After a brief wash, the immobilised ectodomains were incubated for 2 hours at room temperature with pooled sera from malaria-exposed and malaria-naïve individuals diluted 1:1000 in HBST/2%BSA. The plates were washed in HBS/ 0.1% Tween20 (HBST) before incubation with an anti-human immunoglobulin antibody coupled to alkaline phosphatase (Sigma) for one hour at room temperature. After washes in HBST and HBS, wells were incubated with p-nitrophenyl phosphate at 1 mg/ml and optical dentisity measurements (OD) taken at 405nm.

### References

1. Cowman, A.F. & Crabb, B.S. Invasion of red blood cells by malaria parasites. Cell 124, 755-766 (2006).
2. Kauth, C.W. et al. Interactions between merozoite surface proteins 1, 6, and 7 of the malaria parasite Plasmodium falciparum. The Journal of biological chemistry 281, 31517-31527 (2006).
3. Wright, G.J. Signal initiation in biological systems: the properties and detection of transient extracellular protein interactions. Molecular bioSystems 5, 1405-1412 (2009).
4. Durocher, Y., Perret, S. & Kamen, A. High-level and high-throughput recombinant protein production by transient transfection of suspensiongrowing human 293-EBNA1 cells. Nucleic acids research 30, E9 (2002).
5. Bushell, K.M., Sollner, C., Schuster-Boeckler, B., Bateman, A. & Wright, G.J. Large-scale screening for novel low-affinity extracellular protein interactions. Genome research 18, 622-630 (2008).
6. Sim, B.K., Chitnis, C.E., Wasniowska, K., Hadley, T.J. & Miller, L.H. Receptor and ligand domains for invasion of erythrocytes by Plasmodium falciparum. Science (New York, N.Y 264, 1941-1944 (1994).
7. Bushell, K. M., Sollner, C., Schuster-Boeckler, B., Bateman, A., and Wright, G. J. (2008) Large-scale screening for novel low-affinity extracellular protein interactions. Genome Res 18, 622-630.
8. Igakura, T., Kadomatsu, K., Kaname, T., Muramatsu, H., Fan, Q. W., Miyauchi, T., Toyama, Y., Kuno, N., Yuasa, S., Takahashi, M., Senda, T., Taguchi, O., Yamamura, K., Arimura, K., and Muramatsu, T. (1998) A null mutation in basigin, an immunoglobulin superfamily member, indicates its important roles in peri-implantation development and spermatogenesis. Dev Biol 194, 152-165.
9. Fadool, J. M., and Linser, P. J. (1993) 5A11 antigen is a cell recognition molecule which is involved in neuronal-glial interactions in avian neural retina. Dev Dyn 196, 252-262.
10. Zucker, S., Hymowitz, M., Rollo, E. E., Mann, R., Conner, C. E., Cao, J., Foda, H. D., Tompkins, D. C., and Toole, B. P. (2001) Tumorigenic potential of extracellular matrix metalloproteinase inducer. Am J Pathol 158, 1921-1928.
11. Yu, X. L., Hu, T., Du, J. M., Ding, J. P., Yang, X. M., Zhang, J., Yang, B., Shen, X., Zhang, Z., Zhong, W. D., Wen, N., Jiang, H., Zhu, P., and Chen, Z. N. (2008) Crystal structure of HAb18G/CD147: implications for immunoglobulin superfamily homophilic adhesion. J Biol Chem 283, 18056-18065.
12. Pushkarsky, T., Zybarth, G., Dubrovsky, L., Yurchenko, V., Tang, H., Guo, H., Toole, B., Sherry, B., and Bukrinsky, M. (2001) CD147 facilitates HIV-1 infection by interacting with virus-associated cyclophilin A. Proc Natl Acad Sci U S A 98, 6360-6365.
13. Watanabe, A., Yoneda, M., Ikeda, F., Terao-Muto, Y., Sato, H., and Kai, C. CD147/EMMPRIN acts as a functional entry receptor for measles virus on epithelial cells. J Virol 84, 4183-4193.
14. Chen, Z., Mi, L., Xu, J., Yu, J., Wang, X., Jiang, J., Xing, J., Shang, P., Qian, A., Li, Y., Shaw, P. X., Wang, J., Duan, S., Ding, J., Fan, C., Zhang, Y., Yang, Y., Yu, X., Feng, Q., Li, B., Yao, X., Zhang, Z., Li, L., Xue, X., and Zhu, P. (2005) Function of HAb18G/CD147 in invasion of host cells by severe acute respiratory syndrome coronavirus. J Infect Dis 191, 755-760.
15. Coste, I., Gauchat, J. F., Wilson, A., Izui, S., Jeannin, P., Delneste, Y., MacDonald, H. R., Bonnefoy, J. Y., and Renno, T. (2001) Unavailability of CD147 leads to selective erythrocyte trapping in the spleen. Blood 97, 3984-3988.
16. Pasini, E. M., Kirkegaard, M., Mortensen, P., Lutz, H. U., Thomas, A. W., and Mann, M. (2006) In-depth analysis of the membrane and cytosolic proteome of red blood cells. Blood 108, 791-801.
17. Bendtsen, J. D., Nielsen, H., von Heijne, G., and Brunak, S. (2004) Improved prediction of signal peptides: SignalP 3.0. J Mol Biol 340, 783-795.
18. Schlegel, J., Redzic, J. S., Porter, C. C., Yurchenko, V., Bukrinsky, M., Labeikovsky, W., Armstrong, G. S., Zhang, F., Isern, N. G., DeGregori, J., Hodges, R., and Eisenmesser, E. Z. (2009) Solution characterization of the extracellular region of CD147 and its interaction with its enzyme ligand cyclophilin A. J Mol Biol 391, 518-535.
19. Sollner, C., and Wright, G. J. (2009) A cell surface interaction network of neural leucine-rich repeat receptors. Genome Biol 10, R99.
20. van der Merwe, P. A., and Barclay, A. N. (1996) Analysis of cell-adhesion molecule interactions using surface plasmon resonance. Curr Opin Immunol 8, 257-261.

### Discussion/Conclusion

In this study, we have used a simple systematic approach based on codon-optimised constructs transiently expressed in mammalian cells to create a resource of recombinant proteins of the *P*. *falciparum* merozoite surface protein repertoire. With the exception of rhoptry proteins, which proved difficult to express, most proteins were successfully produced. This convenient high throughput system does not require complex refolding procedures. Using a cost effective delivery reagent which results in typical transfection efficiencies of 20%, we were able to obtain good expression levels reaching up to 1.4 mg of purified protein from a 50 mL transfection in some cases. This yield could probably be further increased by creating high-secreting stable cell lines. All proteins were expressed as soluble recombinant ectodomains and, where known, were shown to be correctly folded and functional based on their ability to recapitulate known binding events. The carboxy-terminal tag present on the proteins includes a sequence that can be enzymatically monobiotinylated during protein expression and enables the proteins to be quantified and captured in an oriented fashion on streptavidin-coated solid phases. In this study, we used it to create highly avid binding reagents to identify host erythrocyte receptor interactions and we believe that the proteins in this resource will facilitate the discovery of novel erythrocyte receptors for merozoite surface proteins, many of which are still unknown. The same approach could now be used to easily introduce specific mutations within merozoite proteins and observe the effects of naturally occurring variations on protein function. These proteins could also be fed into protein structure initiatives which will eventually aid the rational design of novel therapeutic drugs. Despite decades of research, malaria continues to be a global health problem and the emergence and rapid spread of drug-resistant strains makes the development of novel therapeutics an urgent research priority. One strategy has been to develop a vaccine based on targeting the merozoite since this form of the parasite is exposed to the host humoral immune system and passive transfer of immunoglobulins to patients with clinical malaria can reduce parasitaemia and resolve symptoms. Many of the current leading vaccine targets are proteins known to be located on the exposed surface of the merozoite. However, the difficulty in producing these proteins in a soluble recombinant form has often led to targets being selected on criteria such as high-level expression in a convenient expression system rather than producing correctly folded antigenically-active proteins that make the most effective vaccines. Because it is increasingly likely that an effective anti-malarial vaccine will not consist of a single protein but will be a multi-component vaccine, we believe that the resource described in this study will represent a significant step towards this goal. Finally, the demonstration that the Rh5-BSG interaction is essential for erythrocyte invasion now provides new opportunities for novel therapeutic intervention strategies. This could include a modification of the AVEXIS assay to identify small molecule inhibitors of the Rh5-BSG interaction or the humanisation of the MEM-M6/1 and MEM-M6/6/ antibodies.

### Figure legends

Figure 1. Expression of recombinant secreted and cell surface merozoite proteins from *P*. *falciparum.* Expression of biotinylated recombinant merozoite proteins was assessed by western blot. The expected molecular weight for each recombinant protein is indicated in brackets above each column.

Figure 2. Functional activity and immunogenicity of recombinant *P. falciparum* merozoite proteins. (A) Recombinant biotinylated PfEBA-175 (top panel) and PfEBA-140 (bottom panel) immobilized on fluorescent streptavidin-coated beads bound to untreated erythrocytes (thick solid grey line). Binding was blocked by pre-treating the erythrocytes with neuraminidase (thin grey line) or (for PfEBA-175) pre-incubating erythrocytes with an anti-GYPA monoclonal antibody (dotted line). Negative controls were Cd4d3+4-coated beads (thin solid black line).

Figure 3. Demonstration that MSP1 and MSP7 are correctly folded and functional. The interaction between recombinant PfMSP-1 and PfMSP-7 was detected in both bait-prey orientations by screening the whole library with PfMSP1 and PfMSP7 preys using the AVEXIS assay. Baits labeled with an asterisk were below threshold levels required for the assay.

Figure 4. Immunogenicity of the recombinant *P*. *falciparum* merozoite surface antigens. The immunogenicity of the recombinant proteins was systematically compared using sera pooled from adult patients unexposed to malaria (naïve sera, open bars) or living within malaria-endemic regions (immune sera, grey bar). Recombinant proteins largely contained heat-labile (conformational) epitopes as seen by the reduced response of immune sera to heat denatured antigen (black bar).

Figure 5. AVEXIS identifies two splice variants of the erythrocyte surface protein BASIGIN as a receptor for *P*. *falciparum* Rh5. (A) Rh5 interacted with both long and short isoforms of BASIGIN but no other erythrocyte receptor protein when screened as either a prey (left panel) or a bait (right panel). Error bars indicate the standard deviation from three replicates. (B) Schematic showing the domain architecture of Rh5 and the BSG isoforms. Rh5 is a secreted protein and contains a region of sequence homology to other Rh-family members indicated by the red box. The long and short BSG isoforms contain three and two IgSF domains respectively, numbered 0 to 2 according to convention. Signal peptides are indicated by an unfilled rectangle and putative N-linked glycosylation sites by lollipops.

Figure 6. Biophysical characterisation of the Rh5-BSG interaction using surface plasmon resonance. (A) Equilibrium binding analysis. Serial dilutions of purified PfRh5Cd4d3+4-6H were injected (solid bar) through flow cells with 325RU of BSG-SCd4d3+4bio or 150RU of Cd4d3+4 (control) for 200 seconds until equilibrium was reached (inset). Reference-subtracted binding data were plotted as a binding curve and a KD of ∼1.1 µM was calculated using non-linear regression fitting of a simple Langmuir binding isotherm to the data (solid line). (B) Kinetic binding analysis. The indicated concentrations of PfRh5Cd4d3+4-6H were injected over immobilised BSG-S surface at 100 µl/min. The binding curves were globally fitted to a 1:1 binding model (red line).

Figure 7a. Soluble recombinant ectodomains of BSG-S and BSG-L potently reduce the efficiency of *P*. *falciparum* erythrocyte invasion. Purified pentameric ectodomains of the short (squares) and long (circles) forms of BSG were added at the indicated concentrations to an in vitro P. falciparum invasion assay using the Dd2 (A) and 3D7 (B) strains. The proteins reduced invasion efficiency relative to a control (CD4-COMP (triangles)).

Figure 7b. Soluble BSG potently block erythrocyte invasion across multiple strains. (a) Erythrocyte invasion was inhibited by purified pentamerised BSG-S-Cd4d3+4-COMP-His ectodomains but not by the two non-binding BSG-S domains added individually or Cd4d3+4-COMP-His (control); strain = Dd2. (b) Cross-strain inhibition of invasion using pentamerised BSG-S.

Figure 8a. Mouse monoclonal antibodies to human BSG block the invasion of *P*. *falciparum* into human erythrocytes. Purified monoclonal antibodies MEM-M6/1 (circles) and MEM-M6/6 (squares) were added at the indicated concentrations to an *in vitro P. falciparum* invasion assay using the 3D7 strain. The proteins reduced invasion efficiency relative to an isotype-matched negative control (diamonds).

Figure 8b. Anti-BSG antibodies potently block erythrocyte invasion. (A) Anti-BSG monoclonal antibodies, TRA-1-85 and MEM-M6/6, potently inhibited invasion of erythrocytes; strain = 3D7. (B) MEM-M6/6 concentrations ≥10 µg/ml prevented all detectable invasion by microscopic observation of cultures; strain = 3D7. (C, D) MEM-M6/6 inhibited invasion of synchronised *P*. falciparum culture-adapted lines (C) and unsynchronised field isolates (D).

**Table 1**

| **Protein name** | **Accession #** | **Region expressed** | **Length** | **N-Gly sites** | **Expression level (µg/ml)** |
|---|---|---|---|---|---|
| MSP1 | PFI1475w | V20-S1701 | 1682 | 13 | 1.25 |
| MSP2 | PFB0300c | I20-N246 | 227 | 4 | 50 |
| MSP4 | PFB0310c | Y29-S253 | 225 | 2 | 25 |
| MSP5 | PFB0305c | N22-S251 | 230 | 4 | 5 |
| MSP10 | PFF0995c | H27-S503 | 477 | 9 | 2.5 |
| Pf12 | PFF0615c | H26-S323 | 298 | 7 | 0.62 |
| Pf38 | PFE0395c | Q22-S328 | 307 | 4 | 1.25 |
| Pf92 | PF13_0338 | A26-S770 | 745 | 15 | 0.03 |
| Pf113 | PF14_0201 | Y23-K942 | 920 | 9 | 0.62 |
| ASP | PFD0295c | A20-S708 | 689 | 10 | 0.39 |
| RAMA | AAQ89710 | Y17-K838 | 821 | 10 | 0.15 |
| EBA140 | MAL13P1.60 | I26-P1135 | 1110 | 11 | 0.004 |
| EBA175 | MAL7P1.176 | A21-P1424 | 1404 | 18 | 0.015 |
| EBA181 | PFA0125c | I27-S1488 | 1462 | 17 | 0.015 |
| EBL1 | PF13_0115 | K22-N2584 | 2563 | 29 | <0.002 |
| AMA1 | PF11_0344 | Q25-T541 | 517 | 6 | 25 |
| MTRAP | PF10_0281 | I23-K432 | 410 | 10 | 0.62 |
| MSP3 | PF10_0345 | K26-H354 | 328 | 4 | 12.5 |
| MSP6 | PF10_0346 | Y17-N371 | 355 | 3 | 0.156 |
| H101 | PF10_0347 | Q23-N424 | 402 | 6 | 0.39 |
| H103 | PF10_0352 | K27-Y405 | 379 | 4 | 1.56 |
| MSP7 | PF13_0197 | T28-M351 | 324 | 2 | 6.24 |
| Pf41 | PFD0240c | K21-S378 | 358 | 6 | 6.24 |
| RAP1 | PF14_0102 | I23-D782 | 760 | 7 | <0.002 |
| RAP2 | PFE0080c | D22-L398 | 387 | 2 | <0.002 |
| RAP3 | PFE0075c | N23-K400 | 378 | 4 | <0.002 |
| RhopH1 | PFC0110w | K21-H1416 | 1396 | 10 | <0.002 |
| RhopH2 | PFI1445w | L20-S1378 | 1359 | 13 | <0.002 |
| RhopH3 | PFI0265c | K25-L897 | 873 | 4 | 0.312 |
| Rh1 | PFD0110w | Q24-T666 | 643 | 7 | <0.002 |
| Rh2b* | PF13_0198 + MAL13P1.176 | H25-Y75 + M1-S953 | 1003 | 13 | <0.002 |
| Rh4 | PFD1150c | I27-T1148 | 1122 | 20 | <0.002 |
| Rh5 | PFD1145c | F25-Q526 | 502 | 4 | 0.078 |
| PTRAMP | PFL0870w | N25-S306 | 282 | 7 | <0.002 |
| SPATR | PFB0570w | E22-C250 | 229 | 2 | 12.5 |
| TLP | PFF0800w | E24-P1306 | 1283 | 27 | 0.195 |
| Pf34 | PFD0955w | N25-S306 | 282 | 2 | 6.24 |
| PF14_0344 | PF14_0344 | A20-N993 | 974 | 13 | 0.39 |
| PF10_0323 | PF10_0323 | R25-R52 | 28 | 0 | 1.56 |
| RON3 | PFL2505c | N22-N249 | 228 | 1 | <0.002 |
| PFF0335c | PFF0335c | V23-K299 | 277 | 3 | 12.5 |
| AARP | PFD1105w | K18-P191 | 174 | 5 | 0.312 |
| MSP3.4 | PF10_0348 | N26-K697 | 672 | 11 | 0.2 |
| MSP3.8 | PF10_0355 | Y23-N762 | 740 | 10 | 0.39 |
| MSRP1 | PF13_0196 | Y22-T379 | 358 | 4 | 1.56 |
| MSRP2 | MAL13P1.174 | K24-T280 | 257 | 5 | 0.312 |
| MSRP3 | PF13_0193 | Q24-S298 | 275 | 3 | 1.56 |
| RON6 | PFB0680w | F16-T949 | 934 | 15 | 0.01 |
| Pf12p | PFF0620c | Y21-T349 | 329 | 3 | 0.15 |
| MSP9 | PFL1385c | N24-S742 | 719 | 9 | 0.03 |
| GAMA | PF08_0008 | L22-P710 | 689 | 9 | 0.78 |
| PF11_0373 | PF11_0373 | L19-G656 | 638 | 20 | 0.78 |
| PF14_0293 | PF14_0293 | N25-S968 | 944 | 23 | 0.01 |

| | | | | | |
|---|---|---|---|---|---|
| * Because of the absence of clear signal peptide in the Rh2b protein sequence (MAL13P1.176), the N-terminus of the Rh2a sequence (PF13_0198) was added at the amino-terminus. | | | | | |

**Table 1.** A list of recombinant merozoite proteins from *P*. *falciparum.*
The first and last amino-acid of the ectodomain region expressed in HEK293E cells is shown for each protein, along with the number of potential N-linked glycosylation sites that were modified and the level of expression as assessed by ELISA.

## Claims

1. An antibody which binds to CD147, for use in the prevention and/or treatment of *Plasmodial* infection and/or malarial disease.

2. The antibody for use according to claim 1, wherein the antibody is Metuximab.

3. The antibody for use according to claim 1, wherein the antibody is an antibody that is capable of competing with MEM-M6/6 or TRA-1-85 for binding to CD147.

4. The antibody for use according to claim 1, wherein the antibody is a humanised version of either of the antibodies MEM-M6/6 or MEMM6/1.

5. The antibody for use according to claim 1, wherein the antibody is a humanised anti-CD147 antibody.

6. The antibody for use according to claim 1, wherein the antibody is the radiolabeled F(ab)'2 metuximab-l131.

## Patentansprüche

1. Antikörper, der an CD147 bindet, zur Verwendung bei der Prävention und/oder Behandlung einer Plasmodium-Infektion und/oder Malaria-Krankheit.

2. Antikörper zur Verwendung nach Anspruch 1, wobei der Antikörper Metuximab ist.

3. Antikörper zur Verwendung nach Anspruch 1, wobei der Antikörper ein Antikörper ist, der in der Lage ist, mit MEM-M6/6 oder TRA-1-85 um die Bindung an CD147 zu konkurrieren.

4. Antikörper zur Verwendung nach Anspruch 1, wobei der Antikörper eine humanisierte Version von einem der Antikörper MEM-M6/6 oder MEMM6/1 ist.

5. Antikörper zur Verwendung nach Anspruch 1, wobei der Antikörper ein humanisierter Anti-CD147-Antikörper ist.

6. Antikörper zur Verwendung nach Anspruch 1, wobei der Antikörper das radioaktiv markierte F(ab)'2 Metuximab-l131 ist.

## Revendications

1. Anticorps qui se lie à CD147, destiné à être utilisé dans la prévention et/ou le traitement d'une infection plasmodiale et/ou du paludisme.

2. Anticorps à utiliser selon la revendication 1, dans lequel l'anticorps est le métuximab.

3. Anticorps à utiliser selon la revendication 1, dans lequel l'anticorps est un anticorps qui est capable de rivaliser avec MEM-M6/6 ou TRA-1-85 pour se lier à CD147.

4. Anticorps à utiliser selon la revendication 1, dans lequel l'anticorps est une version humanisée de l'un ou l'autre des anticorps MEM-M6/6 ou MEMM6/1.

5. Anticorps à utiliser selon la revendication 1, dans lequel l'anticorps est un anticorps anti-CD147 humanisé.

6. Anticorps à utiliser selon la revendication 1, dans lequel l'anticorps est le métuximab-I131 F(ab)'2 radiomarqué.


### SEQUENCE LISTING

<110> Genome Research Limited
<120> Immunogenic compositions and Expression systems
<130> 16199.26WO
<150> GB1016969.6
   <151> 2010-10-08
<160> 1
<170> PatentIn version 3.5
<210> 1
   <211> 502
   <212> PRT
   <213> Plasmodium falciparum
<400> 1
   Phe Glu Asn Ala Ile Lys Lys Thr Lys Asn Gln Glu Asn Asn Leu Ala
   1               5                   10                  15
   Leu Leu Pro Ile Lys Ser Thr Glu Glu Glu Lys Asp Asp Ile Lys Asn
               20                  25                  30
   Gly Lys Asp Ile Lys Lys Glu Ile Asp Asn Asp Lys Glu Asn Ile Lys
           35                  40                  45
   Thr Asn Asn Ala Lys Asp His Ser Thr Tyr Ile Lys Ser Tyr Leu Asn
       50                  55                  60
   Thr Asn Val Asn Asp Gly Leu Lys Tyr Leu Phe Ile Pro Ser His Asn
   65                  70                  75                  80
   Ser Phe Ile Lys Lys Tyr Ser Val Phe Asn Gln Ile Asn Asp Gly Met
                   85                  90                  95
   Leu Leu Asn Glu Lys Asn Asp Val Lys Asn Asn Glu Asp Tyr Lys Asn
               100                 105                 110
   Val Asp Tyr Lys Asn Val Asn Phe Leu Gln Tyr His Phe Lys Glu Leu
           115                 120                 125
   Ser Asn Tyr Asn Ile Ala Asn Ser Ile Asp Ile Leu Gln Glu Lys Glu
       130                 135                 140
   Gly His Leu Asp Phe Val Ile Ile Pro His Tyr Thr Phe Leu Asp Tyr
   145                 150                 155                 160
   Tyr Lys His Leu Ser Tyr Asn Ser Ile Tyr His Lys Ser Ser Thr Tyr
                   165                 170                 175
   Gly Lys Cys Ile Ala Val Asp Ala Phe Ile Lys Lys Ile Asn Glu Ala
               180                 185                 190
   Tyr Asp Lys Val Lys Ser Lys Cys Asn Asp Ile Lys Asn Asp Leu Ile
           195                 200                 205
   Ala Thr Ile Lys Lys Leu Glu His Pro Tyr Asp Ile Asn Asn Lys Asn
       210                 215                 220
   Asp Asp Ser Tyr Arg Tyr Asp Ile Ser Glu Glu Ile Asp Asp Lys Ser
   225                 230                 235                 240
   Glu Glu Thr Asp Asp Glu Thr Glu Glu Val Glu Asp Ser Ile Gln Asp
                   245                 250                 255
   Thr Asp Ser Asn His Ala Pro Ser Asn Lys Lys Lys Asn Asp Leu Met
               260                 265                 270
   Asn Arg Ala Phe Lys Lys Met Met Asp Glu Tyr Asn Thr Lys Lys Lys
           275                 280                 285
   Lys Leu Ile Lys Cys Ile Lys Asn His Glu Asn Asp Phe Asn Lys Ile
       290                 295                 300
   Cys Met Asp Met Lys Asn Tyr Gly Thr Asn Leu Phe Glu Gln Leu Ser
   305                 310                 315                 320
   Cys Tyr Asn Asn Asn Phe Cys Asn Thr Asn Gly Ile Arg Tyr His Tyr
                   325                 330                 335
   Asp Glu Tyr Ile His Lys Leu Ile Leu Ser Val Lys Ser Lys Asn Leu
               340                 345                 350
   Asn Lys Asp Leu Ser Asp Met Thr Asn Ile Leu Gln Gln Ser Glu Leu
           355                 360                 365
   Leu Leu Thr Asn Leu Asn Lys Lys Met Gly Ser Tyr Ile Tyr Ile Asp
       370                 375                 380
   Thr Ile Lys Phe Ile His Lys Glu Met Lys His Ile Phe Asn Arg Ile
   385                 390                 395                 400
   Glu Tyr His Thr Lys Ile Ile Asn Asp Lys Thr Lys Ile Ile Gln Asp
                   405                 410                 415
   Lys Ile Lys Leu Asn Ile Trp Arg Thr Phe Gln Lys Asp Glu Leu Leu
               420                 425                 430
   Lys Arg Ile Leu Asp Met Ser Asn Glu Tyr Ser Leu Phe Ile Thr Ser
           435                 440                 445
   Asp His Leu Arg Gln Met Leu Tyr Asn Thr Phe Tyr Ser Lys Glu Lys
       450                 455                 460
   His Leu Asn Asn Ile Phe His His Leu Ile Tyr Val Leu Gln Met Lys
   465                 470                 475                 480
   Phe Asn Asp Val Pro Ile Lys Met Glu Tyr Phe Gln Thr Tyr Lys Lys
                   485                 490                 495
   Asn Lys Pro Leu Thr Gln
               500